**Europäisches Patentamt**

**European Patent Office**

**Office européen des brevets**

(19)

(11) Veröffentlichungsnummer : **0 120 375**
**B1**

(12)

# EUROPÄISCHE PATENTSCHRIFT

(45) Veröffentlichungstag der Patentschrift :
30.12.86

(51) Int. Cl.⁴ : **C 12 M   1/40**, **A 23 C  21/02**

(21) Anmeldenummer : **84102574.5**

(22) Anmeldetag : **09.03.84**

(54) **Verfahren und Vorrichtung zur Behandlung von Trüben, insbesondere zur Hydrolyse des Milchzuckers in Molke.**

(30) Priorität : 23.03.83 DE 3310430

(43) Veröffentlichungstag der Anmeldung :
03.10.84 Patentblatt 84/40

(45) Bekanntmachung des Hinweises auf die Patenterteilung : 30.12.86 Patentblatt 86/52

(84) Benannte Vertragsstaaten :
AT BE CH DE FR GB IT LI NL

(56) Entgegenhaltungen :
EP-A- 0 049 576
GB-A- 2 078 227

(73) Patentinhaber : Röhm GmbH
Kirschenallee Postfach 4242
D-6100 Darmstadt 1 (DE)

(72) Erfinder : Ottofrickenstein, Hans
In der Kirchtanne 6
D-6100 Darmstadt-Eberstadt (DE)
Erfinder : Plainer, Hermann, Dr.
Am Wembach 15
D-6107 Reinheim 1 (DE)
Erfinder : Sprössler, Bruno, Dr.
Auf der Schmelz
D-6101 Rossdorf (DE)
Erfinder : Uhlig, Helmut, Dr.
Auf dem Wingert 4
D-6101 Rossdorf 1 (DE)

## Beschreibung

### Gebiet der Erfindung

Die Erfindung betrifft ein Verfahren zur Behandlung einer flüssigen, feinteilige Feststoffe enthaltenden Trübe mit einem körnigen Behandlungsmittel, welches in einem von der Trübe durchströmten Festbett angeordnet ist. Insbesondere betrifft die Erfindung ein Verfahren zur Hydrolyse des Milchzuckers in Molke mittels einer Lactase, die auf Trägerteilchen immobilisiert ist. Die mit der Lactase beladenen Trägerteilchen sind in einem meist rohrförmigen Reaktor als Festbett angeordnet. Dadurch wird die Substrathemmung unterdrückt, die in einem Wirbelbettkatalysator den Umsatz des Milchzuckers begrenzen würde.

### Stand der Technik

Die Hydrolyse des Milchzuckers in Molke ist ein typisches Verfahren der hier betrachteten Art.

Die kontinuierliche Milchzuckerhydrolyse mittels einer Lactase, die adsorptiv an perlförmige Trägerteilchen gebunden ist, ist in der DE-OS 28 39 737 beschrieben. Das Verfahren läßt sich nur dann über mehrere Tage kontinuierlich durchführen, wenn ein Molke-Permeat eingesetzt wird, aus dem die natürliche Trübung durch Ultrafiltration entfernt worden ist.

Beim Verfahren gemäß DE-OS 31 22 231 wird die Molke angesäuert, erhitzt und zentrifugiert, um Trübstoffe zu entfernen. Trotzdem ist bei diesem Verfahren eine tägliche Reinigung des Enzymkatalysators unerläßlich, denn selbst nach der Klärung verbleiben noch so viele kolloidal gelöste Eiweißstoffe in der Molke, daß sich auf den Trägerteilchen ein Überzug abscheidet, der die Aktivität der immobilisierten Lactase beeinträchtigt. Zur Reinigung wird der als Festbett angeordnete Enzymkatalysator aufgewirbelt und mit einer Proteaselösung durchspült, die den Proteinüberzug abbaut und die Lactase reaktiviert.

Dieses Verfahren ist wegen der Vorbehandlung der Molke durch Erhitzen und Zentrifugation aufwendig und wenig wirtschaftlich. Setzt man jedoch ungeklärte Molke mit einer Trübung über 100 NTU bei diesem Verfahren ein, so verwandelt sich die Festbettschüttung des Enzymkatalysators allmählich in einen zusammenhängenden Pfropfen, der sich auch durch Umkehrung der Strömungsrichtung nicht aufwirbeln und reinigen läßt. Diese Erscheinung wird vor allem bei häufiger Wiederverwendung des Katalysators beobachtet.

### Aufgabe und Lösung

Es war die Aufgabe zu lösen, ein einfach durchführbares technisches Verfahren zu finden, das die kontinuierliche Behandlung einer Trübe ohne vorausgehende Verfahrensschritte zur Klärung der Trübe ermöglicht. Im engeren Sinne bestand die Aufgabe darin, ein in Molkereien einfach durchführbares technisches Verfahren zu finden, das die kontinuierliche Hydrolyse von Milchzucker in unfiltrierter Molke durchzuführen gestattet und eine lange Lebensdauer des Enzymkatalysators, d. h. eine große Zahl von Hydrolyse/Reinigungs-Zyklen gewährleistet.

Die Aufgabe wurde durch das Verfahren gemäß Anspruch 1 und die Vorrichtung gemäß Anspruch 10 gelöst. Für die Verarbeitung ganzer Molke mit einer Trübung über 100 NTU wurde eine Lösung der gestellten Aufgabe in dem Verfahren gemäß Anspruch 6 und in der Durchführung des Verfahrens in der Vorrichtung gemäß dem Anspruch 14 gefunden.

### Vorteile der Erfindung

Bei der Durchströmung des Festbettes durch die Trübe läßt sich die Ablagerung der feinteiligen Feststoffe in den Zwischenräumen in dem Festbett des körnigen Behandlungsmittels nicht vermeiden. Dadurch wird die Berührungsfläche zwischen den Teilchen des Behandlungsmittels allmählich kleiner und die Wechselwirkung mit dem Behandlungsmittel geringer.

Im Falle der Hydrolyse ganzer Molke in einem enzymatischen Festbettkatalysator tritt unvermeidbar nach einer gewissen Betriebsdauer, die von dem Trübungsgehalt der Molke und von der Beschaffenheit der Enzymkatalysatorteilchen abhängt, starker Wirksamkeitsverlust ein, verbunden mit einem Anstieg des Durchströmungswiderstandes des Festbettes und verursacht durch die Ablagerung der hauptsächlich aus Milcheiweißpartikeln bestehenden Trübstoffe aus der Molke in den Zwischenräumen zwischen den Katalysatorteilchen. Solange der Katalysator noch ziemlich frisch ist, lassen sich durch Aufwirbeln des Festbettes mit einer wäßrigen Reinigungsflüssigkeit im Gegenstrom die Ablagerungen weitgehend entfernen und die Aktivität des Katalysators wiederherstellen. Nach mehreren Reinigungszyklen gelingt dies immer weniger, so daß man zu immer kürzeren Betriebszeiten zwischen zwei Reinigungszyklen übergehen muß. Auch sinkt die Aktivität nach jeder Reinigung weiter ab. Die Gefahr einer mikrobiellen Kontamination steigt. Das gleiche gilt für andere enzymatische Verfahren in trüben Substratflüssigkeiten.

Beim Verfahren der Erfindung wird das Festbett, das durch die Ablagerungen zu einem zusammenhängenden Pfropfen verfestigt sein kann, durch den Flüssigkeitsdruck der Reinigungsflüssigkeit in dem Reaktionsraum wie ein Kolben im Zylinder vorangetrieben und gegen Zerteilungsmittel gedrückt, die das Festbett in Bruchstücke zerlegen. Sobald das gesamte Festbett in dieser Weise zerteilt ist, werden die Bruchstücke solange im Fließbett wirbelnd bewegt, bis sie weiter zu einzelnen fluidisierten Einzelteilchen zerfallen und von anhaftenden Ablagerungen befreit sind. Wenn nicht regelmäßig, am besten täglich, das Festbett mit mechanischen Mitteln gereinigt wird, sammeln sich darin soviel Trübpartikel an, daß die Belegung des Trägers in manchen Fällen irreversibel wird. Die Molkehydrolyse gehört zu diesen Fällen.

Das Verfahren gestattet ein günstiges Verhältnis von Behandlungs- zu Reinigungszeiten und dadurch eine hohe Produktivität.

Die Lebensdauer des Behandlungsmittels wird erhöht, weil seine Aktivität bei jedem Zyklus in einem hohen Maße wiederhergestellt wird. Es würde einen weitaus größeren Aufwand an Zeit und Mitteln bedeuten, wenn das Behandlungsmittel nach jedem Zyklus aus dem Reaktor entnommen und in einer getrennten Vorrichtung regeneriert würde.

Diese Vorteile werden mit geringem zusätzlichem apparativem Aufwand erzielt. Vorrichtungen, um die Reinigungsflüssigkeit im Kreislauf durch den Reaktionsraum zu leiten, werden auch bei der bekannten Arbeitsweise benötigt. Sie müssen für das Verfahren der Erfindung gegebenenfalls zur Erzeugung eines höheren Flüssigkeitsdruckes ausgelegt sein. In dem Reaktor selbst sind als zusätzliche apparative Bestandteile nur Zerteilungsmittel erforderlich, die im einfachsten Fall unbeweglich eingebaut sind, vorzugsweise aber mit einem äußeren Antrieb vorzugsweise rotierend bewegt werden.

Das körnige Behandlungsmittel kann jedes feinteilige, feste Material sein, mit dem ein dünnflüssiges Substrat in einem Festbett behandelt werden kann. Typische Fälle sind

a) Ionenaustauscher zur Bindung von Säuren oder Basen, die in dem flüssigen Substrat gelöst sind ;

b) Feststoffkatalysatoren für die heterogene Flüssigphasenumsetzung des flüssigen Substrats oder darin gelöster Inhaltsstoffe ;

c) Adsorptionsmittel zur Bindung von gelösten Inhaltsstoffen aus dem flüssigen Substrat ;

d) Adsorptionsmittel zur chromatographischen Trennung eines Gemisches von Inhaltsstoffen des flüssigen Substrats ;

e) auf Trägerteilchen immobilisierte Enzyme zur enzymatischen Umsetzung von gelösten Inhaltsstoffen des flüssigen Substrats ;

f) auf Trägerteilchen immobilisierte biologisch aktive Stoffe für die Affinitätschromatographie ;

g) inaktive Füllstoffe zur Filtration der festen Trübstoffpartikel aus dem flüssigen Substrat.

Verfahren, in denen derartige Behandlungsmittel eingesetzt werden, gegebenenfalls unter Verwendung klarer oder schwach getrübter flüssiger Subtrate, sind bekannt. Daher kann hier auf eine eingehende Darstellung der verwendbaren körnigen Behandlungsmittel verzichtet werden.

Für alle körnigen Behandlungsmittel gilt, daß ihre Teilchen in dem Sinne fest sein sollen, daß sie sich unter dem Strömungsdruck des flüssigen Substrats nicht so stark verformen, daß sich die durchströmten Zwischenräume schließen. Der Durchmesser der Teilchen kann zwischen 0,05 und 3 mm liegen, insbesondere zwischen 0,2 und 1 mm. Eine möglichst kugelige Gestalt der Teilchen ist von Vorteil. Daher sind durch Perlpolymerisation herstellbare Kunststoffteilchen besonders geeignet, jedoch können auch Körnchen aus mineralischen bzw. keramischen Werkstoffen, gepreßte oder granulierte Pulver, gesinterte oder verglaste Teilchen, Metallkügelchen u. dergl. eingesetzt werden. Eine besondere Rolle spielen in vielen Fällen poröse bzw. makroporöse Teilchen, wie im einzelnen bei den Enzymträgerteilchen besprochen.

Die für die Behandlung des flüssigen Substrats benötigten Aktivstoffe können den Körper der Teilchen des Behandlungsmittels selbst bilden oder auf der äußeren und/oder inneren Oberfläche eines sonst inerten Teilchens gebunden sein.

Der für die bevorzugte Ausführungsform der Erfindung, nämlich die Molkehydrolyse, einzusetzende Enzymkatalysator wird nachfolgend im einzelnen beschrieben.

Der Enzymkatalysator enthält als aktiven Bestandteil eine Lactase, vorzugsweise aus Mikroorganismen, z. B. aus Aspergillus-Arten, wie Aspergillus oryzae, Aspergillus niger, aus Hefen, wie Saccharomyces Kluyveromyces oder aus Bakterien, wie Bac. stearothermophylus oder Escherichia coli. Die Lactase ist in üblicher Weise an einen partikelförmigen Träger gebunden. Eine Methode der adsorptiven Bindung von Lactase, insbesondere aus Escherichia coli, an einen hydrophob modifizierten Träger ist in der DE-OS 2 839 737 beschrieben. Zur covalenten Bindung können Oxiranharzperlen verwendet werden, and die das Enzym durch Reaktion mit Glycidylgruppen gebunden wird. Geeignete Oxiranperlen sind in DE-PS 2 237 316 und DE-PS 2 722 751 beschrieben. Man kann auch die Lactase zunächst adsorptiv binden und danach .eine Vernetzung mit einer Kupplungskomponente, wie Glutardialdehyd, vornehmen. Weiterhin können immobilisierte Zellen von lactasebildenden Mikroorganismen als Enzymkatalysator verwendet werden.

Die Aktivität der immobilisierten Lactase sollte nicht unter 100 Lactase-U/g Feuchtträger liegen. Der bevorzugte Aktivitätsbereich liegt bei 200 bis 400 U/g. 1 Lactase-U ist definiert als jene Menge feuchter Enzymträger, die unter Standarbedingungen (30 °C, pH = 4,5) aus einer 5 %igen Lactaselösung 1 $\mu$m/min Glucose freisetzt.

Als Träger für Enzyme im allgemeinen und die Lactase im besonderen eignet sich eine Vielzahl von organischen oder anorganischen Stoffen mit makroporöser Struktur. Darunter werden Stoffe mit einem Porenvolumen von mindestens 1 ml/g und einer mittleren Porenweite von 1 bis 500 nm verstanden. Träger mit einer hydrophilen, insbesondere nichtionischen Matrix sind bevorzugt. Der Durchmesser der Teilchen kann zwischen 0,1 und 1 mm liegen ; bevorzugt 0,2 bis 1,0 mm. Eine möglichst kugelige Gestalt der Einzelteilchen ist von Vorteil. Daher sind durch Perlpolymerisation herstellbare makroporöse Polymerisate besonders geeignet.

Beispiele anorganischer Träger sind keramische Kügelchen, makroporöses Glas oder Aluminiumoxid. Um Bindungsplätze für die Lactase zu erzeugen, können diese Träger silanisiert werden, z. B. mittels γ-Aminopropyl-triäthoxysilan, woran sich das Enzym mit Glutardialdehyd kuppeln läßt. Organische Träger können aus Naturstoffen, wie Kork oder Maisspindelgranulat oder makroporösen Kunststoffen, wie Acrylharzen, Polystyrol, Phenol-Formaldehydharzen und dergleichen bestehen. Herstellungsverfahren für solche makroporösen Stoffe sind z. B. in EP-A 58767 beschrieben. Trägerpolymere mit hydrophiler, nichtionischer Matrix werden vor allem durch inverse Perlpolymerisation von Amiden oder Hydroxyalkylestern der Acryl- oder Methacrylsäure erhalten.

Die flüssige Trübe soll, damit sie den Festbettreaktor zu durchströmen vermag, eine Viskosität nicht über 1 000 mPa · s haben. Die erfindungsgemäß eingesetzten Trüben enthalten einen feinteiligen Feststoff, der die Trübung hervorruft. Der Trübungswert, gemessen nach der Methode der Nephelometrie, beträgt mehr als 10 NTU, vorzugsweise mehr als 100 NTU, in der Regel etwa 200 bis 700 NTU. Die Trübungseinheit NTU (nephelometric turbidity unit) wird auch als JTU (Jackson turbidity unit) oder FTU (formazin turbidity unit) bezeichnet. Übliche Nephelometer (z. B. DRT-100 von Fisher Scientific Company) sind bereits auf diese Einheit geeicht. Die Temperatur der Molke kann je nach dem Aktivitätsoptimum der immobilisierten Lactase zwischen 0 und 60 °C liegen.

Obwohl eine nephelometrische Obergrenze für das Verfahren der Erfindung nicht leicht angegeben werden kann, ist es auf die Verarbeitung von « Trüben » im sprachüblichen Sinne beschränkt. Flüssige Suspensionen, Schlämme, Breie und dergleichen, die schon in dünner Schicht lichtundurchlässig sind, gehören ebensowenig zu den erfindungsgemäß verarbeiteten Trüben wie Aufschlämmungen, aus denen sich die Feststoffe nach kurzer Zeit absetzen. Der höchstmögliche Gehalt an trübenden Feststoffen hängt von deren Teilchengröße und Absetzneigung in dem Festbett ab. Auch Trüben mit verhältnismäßig hohen Feststoffgehalten sind verarbeitbar, wenn sich die Trübteilchen aus Festbett nicht in erheblichem Maße absetzen. Typische Trüben, die nach dem Verfahren der Erfindung verarbeitet werden, sind solche, die nach einer Betriebsdauer von mindestens 6, vorzugsweise mindestens 12 bis 24 Stunden oder mehr eine Reinigung des Festbettes erforderlich machen. In extremen Fällen kann eine Reinigung schon nach 1- oder 2-stündiger Betriebsdauer erforderlich werden. Die Trüben enthalten in der Regel 0,1 bis 50 g Trübstoffe pro Liter.

Die Trübstoffe können organischer oder anorganischer Natur und natürlichen oder künstlichen Ursprungs sein. Organische Trübstoffe sind z. B. Abbauprodukte von pflanzlichen, tierischen oder mikrobiellen Organismen, pflanzliche Bestandteile in Maischen und Fruchtsäften, Ausflockungen in tierischem Blut, ungelöste Proteinteilchen in Eiweißhydrolysaten, Schwebstoffe in geklärten Abwässern, Cellulosefasern in Abwässern der Papierindustrie, Textilfasern in Wasch- und Reinigungsflotten und Latexteilchen aus natürlichen oder synthetischen, gegebenenfalls koagulierten Latices. Zu den anorganischen Trübstoffen zählen Tonmineralien, Ruß, Staub- oder Feinschlacken aus Naßentstäubungsanlagen, Erz- und Gesteinspartikel und dergleichen.

In der Mehrzahl der Fälle enthalten die Trüben Wasser bzw. wäßrige Lösungen als flüssiges Medium. In diesem Falle wird auch eine wäßrige Reinigungsflüssigkeit verwendet. Andere flüssige Medien sind z. B. Mineralöl, Benzin, Chlorkohlenwasserstoffe oder andere organische Lösungsmittel. Das flüssige Medium kann neben den suspendierten Feststoffen anorganische oder organische Stoffe in gelöster Form enthalten, wie Salze, Fette, Zucker, Albumine, u. dergl.

Die Erfindung hat besondere Bedeutung für die Verarbeitung von Molke. Die erfindungsgemäß eingesetzte Molke wird in der Molkerei zur Unterscheidung von vorerhitzten und anschließend zentrifugierten Molken oder durch Ultrafiltration gereinigtem Permeat allgemein als « ganze Molke » bezeichnet. Lediglich grobflockige oder krümelige Eiweißteilchen werden vor der Verarbeitung abgetrennt. Die verbleibende ganze Molke, die immer noch 0,7 bis 0,8 % Eiweiß und 4,0 bis 4,9 % Milchzucker enthält, hat eine stabile, durch übliche Filter oder durch Absetzen nicht entfernbare Trübung.

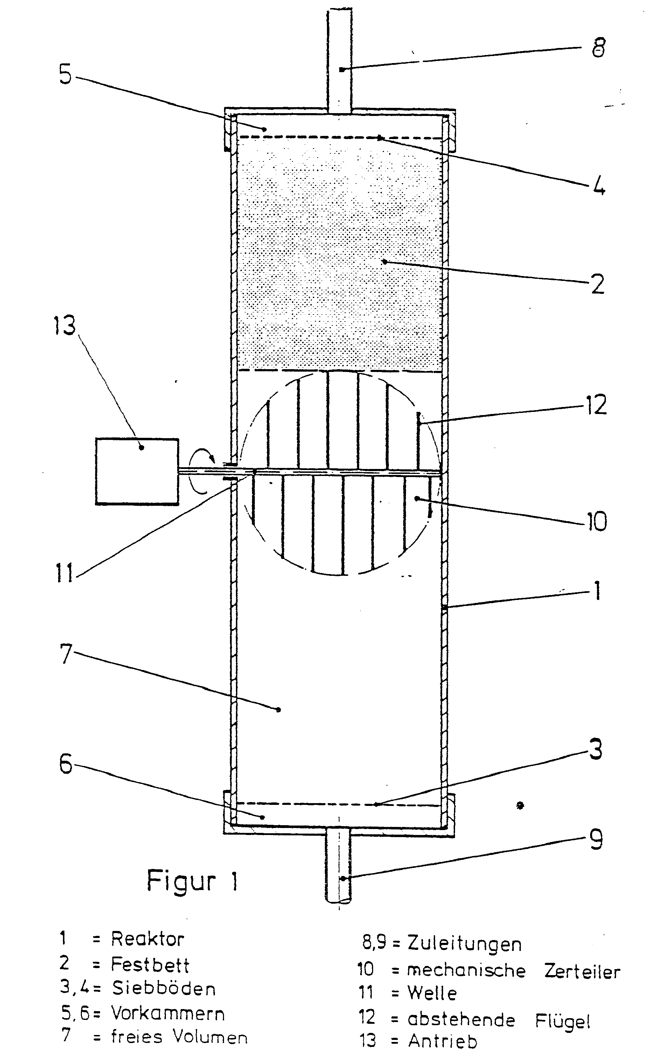

Der Reaktor, der sich zur Durchführung des Verfahrens eignet, wird nachfolgend anhand einer vorteilhaften Ausführungsform, die in Figur 1 als schematisches Schnittbild dargestellt ist, näher beschrieben. Er enthält ein Reaktionsgefäß 1, dessen Innenraum höchstens zur Hälfte, vorzugsweise zu 10 bis 40 Vol.-% mit einem Festbett 2 aus den Teilchen des körnigen Behandlungsmittels angefüllt ist. Der Innenraum ist auf zwei Seiten mit Siebböden 3, 4 von den Vorkammern 5, 6 getrennt. Die Siebböden haben Öffnungen solcher Größe, daß die Teilchen des Behandlungsmittels nicht durchtreten können. Das Reaktionsgefäß ist wenigstens in dem Teil, worin während der Behandlung das Festbett angeordnet ist, vorzugsweise zylindrisch gebaut. Der andere Teil 7 ist während der Behandlung nur mit der zuströmenden Trübe gefüllt. Die Siebböden 3, 4 grenzen die Vorkammern 5, 6 vom Reaktionsraum ab. Das Volumen der Vorkammern soll klein gehalten sein. In die Vorkammern münden Leitungen 8, 9 zum Einleiten und Abführen von Flüssigkeiten oder Gasen. Ein wesentliches Merkmal des Reaktors sind die etwa in der Mitte eingebauten Verteilungsmittel 10. Sofern sie unbeweglich sind, können sie aus einem den Querschnitt des Reaktors oder einen wesentlichen Teil davon überdeckenden Gitter oder Rost aus Stäben bestehen, die in Richtung auf das Festbett 2 mit Spitzen oder Schneiden ausgerüstet sind. Geeignet sind alle Gestaltungen, die das Festbett zu Bruchstücken zerteilen, wenn es durch den Flüssigkeitsdruck dagegen gedrückt wird. Es können auch mehrere Zerteilungsmittel an verschiedenen Stellen des freien Reaktorraumes 7 angeordnet sein.

Bei einer bevorzugten Ausführungsform sind die Zerteilungsmittel in der Weise bewegbar ausgebildet, daß sie eine quer zur Längsachse des Reaktors verlaufende Bewegung ausführen, bzw. eine Bewegung, die eine solche Komponente hat. Wenn zu Beginn der Reinigungsstufe das Festbett gegen die bewegte Zerteilungsvorrichtung getrieben wird, greift diese in das Festbett ein, trennt die Teilchen voneinander oder zerteilt das Festbett zu Bruchstücken, die sich beim Fluidisieren weiter zerlegen. Als Zerteilungsmittel dieser Art eignet sich z. B. eine drehbare Welle 11 mit einer Mehrzahl von abstehenden Flügeln 12. Mit einem Antrieb 13, der nur zu Beginn der Reinigungsstufe betätigt wird, wird die Welle 11 gedreht, wodurch die Flügel 12 in die jeweils oberste Schicht des Festbettes eingreifen. Es versteht sich, daß eine Vielzahl ähnlicher drehend oder pendelnd bewegter Vorrichtungen den gleichen Zweck erfüllen könnte.

Die Verteilungsmittel sind in dem Reaktor in der Regel so angeordnet, daß sie wenigstens im unbewegten Zustand außerhalb des Fließbettes, aber gegebenenfalls dicht über dessen Oberfläche liegen.

## Durchführung des Behandlungsverfahrens

Die Trübe wird so durch das Festbett gefördert, daß sie aus dem freien Reaktorraum 7 in das Festbett 2 eintritt und dieses durch den Siebboden 4 verläßt. Vorzugsweise ist die Strömungsrichtung dabei von unten nach oben gerichtet und die Strömungsgeschwindigkeit der Trübe so hoch, daß die Teilchen des Behandlungsmittels im oberen Bereich des Reaktors zu einem Festbett aufgeschwemmt werden. Wenn die Strömungsgeschwindigkeit geringer ist als die Sinkgeschwindigkeit der Katalysatorteilchen, so ist eine Strömung von oben nach unten vorteilhafter.

Im Falle der Molkehydrolyse richten sich die Hydrolysebedingungen wie Verweilzeit, Temperatur, pH-Wert, Ionenkonzentration usw. weitgehend nach den Eigenschaften des immobilisierten Enzyms. Bei günstiger Wahl der Bedingungen kann ein Hydrolysegrad von 80-90 % über eine Betriebszeit von 12 bis 24 Stunden pro Zyklus aufrechterhalten werden.

## Durchführung der Reinigung

Im Interesse eines gleichmäßigen hohen Umsatzes wird die Reinigungsstufe zweckmäßig begonnen, bevor die Aktivität des Behandlungsmittels merklich nachläßt. Besonders bewährt sich eine Zyklusdauer von 24 Stunden, die sich in 18 bis 22 Stunden Hydrolysezeit und 2 bis 6 Stunden Reinigungszeit aufteilt.

Spätestens wird mit der Reinigungsstufe jeweils dann begonnen, wenn die Aktivität auf weniger als 60 % des Anfangswertes abgenommen oder der Strömungswiderstand des Festbettes auf das 2-fache zugenommen hat.

Zu Beginn der Reinigungsstufe wird der Zustrom frischer Trübe unterbrochen und stattdessen eine Reinigungsflüssigkeit eingeleitet. Zunächst kann ohne Umkehrung der Strömungsrichtung die noch im Reaktor befindliche Trübe durch Wasser oder eine andere substratfreie Flüssigkeit verdrängt werden. Danach wird die Strömungsrichtung umgekehrt und in die Vorkammer 6, aus der bis dahin die behandelte Trübe, z. B. das Molkehydrolysat entnommen wurde, eine wäßrige Reinigungsflüssigkeit unter einem Druck eingeleitet, der das ganze Festbett in dem Reaktor in Bewegung setzt und gegen die Zerteilungsmittel 10 drückt. Sobald das Festbett vollständig an den Zerteilungsmitteln vorbeigelaufen und zerteilt ist, kann der Antrieb der Zerteilungsmittel angehalten werden. Das zu Bruchstücken des Festbettes oder möglichst zu Einzelteilchen zerteilte Behandlungsmittel wird nun zur Reinigung in einem Fließbettzustand gehalten, während ständig die Reinigungsflüssigkeit durch den Reaktor fließt. Die Strömungsrichtung kann dabei mit derjenigen der Trübe bei der Hydrolyse übereinstimmen, jedoch muß dabei die Strömungsgeschwindigkeit so begrenzt werden, daß sich die Katalysatorteilchen nicht an dem Siebboden, durch den die Reinigungsflüssigkeit austritt, erneut zu einem Festbett ansammeln, sondern fluidisiert bleiben. Wenn die Trübe bei der Behandlungsstufe von oben nach unten fließt, kann die Reinigungsflüssigkeit in entgegengesetzter Richtung, also von unten nach oben fließen, da dies in jedem Falle die bevorzugte Strömungsrichtung während der Reinigungsstufe ist. Zur Unterstützung der Verwirbelung können Luftblasen durch die strömende Reinigungsflüssigkeit geleitet werden.

Nach Abschluß der Reinigung kann — vorzugsweise in Strömungsrichtung der Trübe — zwischengespült und danach wieder mit dem Einleiten von Molke begonnen werden.

Die Reinigungsflüssigkeit besteht in der Regel aus der substratfreien Flüssigkeit, die das flüssige Medium der Trübe bildet. Zumindest muß sie damit mischbar sein. In dem bevorzugten Fall der Behandlung einer wäßrigen Trübe besteht die Reinigungsflüssigkeit überwiegend aus Wasser. Reines Wasser kann, wie erwähnt, vor Beginn der Reinigungsphase zur Verdrängung der Trübe und ebenso vor dem Wiederbeginn der Behandlungsstufe zur Verdrängung der Reinigungsflüssigkeit verwendet werden. Auch für den Vorgang der Zerteilung des Festbettes kann einfaches Wasser verwendet werden.

Für die Reinigung des Enzymkatalysators für die Molkehydrolyse ist reines Wasser meistens nicht ausreichend. Man kann zum Abbau der abgelagerten Proteine eine lösliche Protease zusetzen. Stattdessen oder zusätzlich können der Reinigungsflüssigkeit Tenside zugesetzt werden. Ebenso kann man zuerst eine proteasehaltige und danach eine tensidhaltige Reinigungsflüssigkeit einsetzen. Als Tenside eignen sich nichtionische aber auch kationische oder anionisch geladene, wasserlösliche, oberflächenaktive Substanzen, wie Petroleumsulfonate, Polyäthylenoxid-Derivate oder Benzalkonium-Salze in einer Konzentration von z. B. 0,001 bis 0,1 %. Während der mehrere Stunden dauernden Reinigung wird die Reinigungsflüssigkeit vorzugsweise laufend im Kreislauf gepumpt. Die Strömungsgeschwindigkeit im Reaktor wird so eingestellt, daß die Katalysatorteilchen dauernd im fluidisierten Zustand bleiben, aber die abgelösten Proteinteilchen durch den Siebboden mit der abströmenden Reinigungsflüssigkeit ausgetragen werden. Man läßt die Flüssigkeit über einen Filter oder einen Zentrifugalabscheider laufen und führt sie mittels einer Pumpe wieder in den Reaktor ein. Stattdessen kann auch laufend ein Teil der Reinigungsflüssigkeit ausgekreist und durch frische Flüssigkeit ersetzt werden. Ist die Spülflüssigkeit reines Wasser, wird sie vorzugsweise nur einmal durchgepumpt.

Weiterhin hat es sich bewährt, der Reinigungsflüssigkeit ein Desinfektionsmittel zuzusetzen, um den Befall des Reaktors und des Katalysators mit Mikroorganismen zu verhindern. Dies geschieht vorzugsweise nach der Behandlung mit der enzymhaltigen Reinigungsflüssigkeit, jedenfalls aber erst nach dem Fluidisieren der Katalysatorteilchen. Als Desinfektionsmittel eignen sich z. B. Wasserstoffperoxid, Peressigsäure oder quartäre Ammoniumsalze, die gegebenenfalls gleichzeitig Tensidwirkung haben können. Die Desinfektionsmittel werden in einer wirksamen Konzentration eingesetzt und vorzugsweise bei jedem Zyklus gewechselt, um der Ansammlung resistenter Keime vorzubeugen. Das gilt in entsprechender Weise auch für den Einsatz anderer Enzymkatalysatoren und gegebenenfalls bei allen Verfahren, bei denen ein mikrobieller Befall des Behandlungsmittels zu befürchten ist.

Beispiel

Ein Reaktor entsprechend Figur 1 aus durchsichtigem Acrylglas mit einem Innendurchmesser von 8 cm und einer Höhe von 70 cm wird mit 1 kg einer handelsüblichen trägergebundenen Lactase aus Aspergillus oryzae (Handelsbezeichnung Plexazym LA 1, Röhm GmbH) gefüllt. Der Enzymkatalysator hat folgende Eigenschaften :

Perlen, mittl. Durchmesser 0,5 mm
Elektroneutrale Matrix
Aktivität : 250 Lactase-U/g Feuchtträger.

Über dieses Festbett wird bei 35 °C täglich 20 Std. lang Sauermolke aus der Quarkfabrikation geleitet. Die Sauermolke hat folgende Eigenschaften :

Trockengehalt : 6,0-6,5 %
Lactosegehalt : 4-4,5 %
pH = 4,5
Trübung : 650-700 NTU

Keine Sedimentation von Käsestaub in 24 Std. bei Raumtemperatur.

Durch die hohe Durchflußgeschwindigkeit von 50 l/h ist es möglich, bei einer Durchströmung von unten nach oben ein Festbett am oberen Ende der Säule aufzubauen.

Nach jeweils 20 Std. Hydrolysebetrieb wird täglich folgende Reinigungsoperation vorgenommen.

1. Ausspülen der im Reaktor noch vorhandenen Molke mit Wasser,

2. Umkehrung der Strömungsrichtung des Wassers, Zerteilen des Festbettes mit dem mechan. Zerteiler, nötigenfalls mehrfache Wiederholung mit wechselnder Strömungsrichtung,

3. Aufbau eines Fließbettes unter Durchperlen von Luft und Durchleiten von Wasser von unten nach oben mit einer so niedrigen Geschwindigkeit, daß sich die Perlen nicht am Kopf des Reaktors zu einem Festbett sammeln,

4. Entfernung der Trubstoffe durch 1-2 Std. Spülen mit Wasser aus dem Fließbett,

5. Ersatz des Wassers durch ein bekanntes Desinfektionsmittel, z. B.

a) 0,1 %ige Lösung eines quaternären Ammoniumsalzes, wie Benzalkoniumchlorid (Handelsbez. Zephirol, Bayer AG)

b) 0,1 %ige Lösung von $H_2O_2$

c) 0,1 %ige Formaldehydlösung

d) 0,1 %ige Essigsäure

e) 0,1 %ige wäßrige Lösung einer handelsüblichen Protease (Handelsbez. Corolase A, Röhm GmbH).

Die Desinfektionsmittel bzw. die Proteaselösung a) bis e) werden von Tag zu Tag gewechselt. Die Lösungen werden jeweils 30-60 min bei Raumtemperatur im Kreis geführt. Wenn die Keimzahl zu hoch ist, werden zwei verschiedene Desinfektionsmittel hintereinander durchgespült. Fallweise wurde auch kurz auf 50 °C erwärmt.

6. Ausspülen des Desinfektionsmittels mit Wasser ca. 1 Std.

Ergebnis

Der Reaktor wurde 100 Tage lang in dieser Weise betrieben. Der Hydrolysegrad lag während der ganzen Zeit und auch am Ende noch bei etwa 90 % am Beginn des jeweiligen Tages-Zyklus und bei 80-85 % am Ende des Zyklus. Der Träger verblieb während der gesamten Zeit im Reaktor und war jeweils nach Durchführung der Reinigungsoperation gut rieselfähig.

Vergleichsversuch

Es wurde wie oben verfahren, jedoch wurde die mechanische Zerteilung weggelassen.

Ergebnis

Nach dem 6. Zyklus war der Hydrolysegrad auf 60 % abgesunken. Das Festbett konnte nur durch langdauerndes Lufteinblasen, oftmalige Umkehr der Strömungsrichtung noch einmal zerteilt werden.

Nach dem 7. Zyklus war das Festbett verklebt und der Hydrolysegrad auf 30 % abgesunken. Nach Öffnen des Reaktors, Herausnehmen des Trägers und einer 5-stündigen Reinigung in einem gesonderten Behälter, umfassend Zerteilen, Waschen und Desinfizieren wurde die Hydrolyse wieder aufgenommen.

8. bis 11. Zyklus : Ungestörte Hydrolyse, aber abnehmende Hydrolysegrade.

12. Zyklus : Hydrolysegrad auf 40 % abgesunken, Verklebung des Festbettes. Erneute Reinigung des Trägers außerhalb des Reaktors. Das Festbett konnte nicht mehr vollständig in Einzelperlen zerlegt werden. Die Desinfektionslösung erreichte nicht mehr die gesamte Trägeroberfläche.

13.-15. Zyklus : Absinken des Hydrolysegrades, zunehmende Verklebung, zunehmendes Hefewachstum, Druckaufbau.

16. Zyklus : Träger total verklebt, um 5 % Hydrolyse. Eine intensive Reinigung und Desinfektion außerhalb des Reaktors ergab kein rieselfähiges Festbett mehr. Durch eine 1-stündige Behandlung des Trägers bei 50 °C wurde die Hefe abgetötet.

17. Zyklus : Nach 20 Std. Betrieb war das Festbett total verklebt und nicht mehr durchströmbar. Eine Reinigung war nicht mehr möglich. Der Träger mußte verworfen werden.

## Patentansprüche

1. Verfahren zur Behandlung einer flüssigen, feinteilige Feststoffe enthaltenden Trübe mit einem körnigen Behandlungsmittel, welches in einem von der Trübe durchströmten Festbett angeordnet ist, wobei die Durchströmung des Festbettes mit der Trübe periodisch unterbrochen und zur Reinigung des körnigen Behandlungsmittels von feinteiligen Feststoffen, die aus der Trübe stammen, im Gegenstrom zur vorherigen Strömungsrichtung der Trübe eine Reinigungsflüssigkeit durch das Festbett geleitet wird und die Teilchen des körnigen Behandlungsmittels fluidisiert und im Fließbettzustand gereinigt werden, dadurch gekennzeichnet, daß eine Trübe mit einer Trübung über 10 NTU eingesetzt wird, und daß das Festbett bei der Reinigung durch den Flüssigkeitsdruck der Reinigungsflüssigkeit gegen mechanisch wirkende Zerteilungsmittel gedrückt wird, wodurch die Teilchen des Behandlungsmittels voneinander getrennt und zu einem Fließbett fluidisiert werden.

2. Verfahren nach Anspruch 1, dadurch gekennzeichnet, daß die flüssige Trübe und die Reinigungsflüssigkeit überwiegend aus Wasser bestehen.

3. Verfahren nach den Ansprüchen 1 oder 2, dadurch gekennzeichnet, daß die Zerteilungsmittel in solcher Weise bewegt werden, daß sie in das Festbett eingreifen und die Teilchen des Behandlungsmittels voneinander trennen.

4. Verfahren nach den Ansprüchen 1 bis 3, dadurch gekennzeichnet, daß die wäßrige Reinigungsflüssigkeit oberflächenaktive Stoffe enthält.

5. Verfahren nach den Ansprüchen 2 bis 4, dadurch gekennzeichnet, daß vor und nach der Durchströmung des Festbettes mit der wäßrigen Reinigungsflüssigkeit eine Zwischenspülung mit Wasser erfolgt.

6. Verfahren nach den Ansprüchen 1 bis 5, dadurch gekennzeichnet, daß als Trübe eine Molke mit einer Trübung über 100 NTU und als Behandlungsmittel ein Enzymkatalysator, enthaltend eine Lactase, die auf Trägerteilchen immobilisiert ist, eingesetzt werden, und daß das Verfahren der Hydrolyse des Milchzuckers in der Molke dient.

7. Verfahren nach Anspruch 6, dadurch gekennzeichnet, daß die wäßrige Reinigungsflüssigkeit Desinfektionsmittel enthält.

8. Verfahren nach den Ansprüchen 6 oder 7, dadurch gekennzeichnet, daß die wäßrige Reinigungsflüssigkeit ein gelöstes proteolytisches Enzym enthält.

9. Verfahren nach den Ansprüchen 6 bis 8, dadurch gekennzeichnet, daß man einen enzymkatalysator verwendet, der eine Lactase-Aktivität von mindestens 100 U/g aufweist.

10. Festbettreaktor zur Durchführung des Verfahrens gemäß den Ansprüchen 1 bis 9, enthaltend einen zwischen zwei Vorkammern angeordneten Reaktionsraum, der höchstens zur Hälfte mit einem Festbett des körnigen Behandlungsmittels angefüllt und von den Vorkammern durch Siebböden, deren Öffnungen kleiner als die Teilchen des Behandlungsmittels sind, abgetrennt ist, und weiterhin Zu- und Abführungsleitungen, die in die Vorkammern münden, dadurch gekennzeichnet, daß in dem Reaktionsraum außerhalb des Festbettes mechanisch wirkende Zerteilungsmittel angeordnet sind, die auf einem wesentlichen Teil des Querschnittes des Reaktionsraumes wirksam sind.

11. Festbettreaktor nach Anspruch 10, dadurch gekennzeichnet, daß die Zerteilungsmittel aus einem Gitter oder Rost von unbewegbar angeordneten Stäben bestehen, die in Richtung auf das Festbett mit Spitzen oder Schneiden ausgerüstet sind.

12. Festbettreaktor nach Anspruch 10, dadurch gekennzeichnet, daß die Zerteilungsmittel in der Weise bewegbar sind, daß sie mittels eines äußeren Antriebs eine quer zur Längsachse des Reaktors verlaufende Bewegung ausführen können.

13. Festbettreaktor nach Anspruch 10, dadurch gekennzeichnet, daß die Zerteilungsmittel aus einer vorzugsweise quer zur Längsachse des Reaktors angeordneten, drehbaren und antreibbaren Welle mit abstehenden Flügeln bestehen.

14. Festbettreaktor nach den Ansprüchen 10 bis 13, dadurch gekennzeichnet, daß er als körniges Behandlungsmittel ein auf Trägerteilchen immobilisiertes Enzym enthält.

15. Enzymatischer Hydrolysereaktor zur Durchführung des Verfahrens nach den Ansprüchen 6 bis 9, dadurch gekennzeichnet, daß das körnige Behandlungsmittel aus einem Enzymkatalysator, enthaltend eine Lactase, die auf Trägerteilchen immobilisiert ist, besteht.

## Claims

1. Process for treating a turbid liquid containing finely divided solids with a granular treating agent which is arranged in a fixed bed through which the turbid liquid flows, wherein the flow of turbid liquid through the fixed bed is periodically interrupted and, in order to cleanse the granular treating agent of any finely divided solids coming from the turbid liquid, a cleaning liquid is passed through the fixed bed counter to the previous direction of flow of the turbid liquid and the particles of the granular treating agent are fluidized and cleansed in their fluidized state, characterised in that a turbid liquid with a turbidity of more than 10 NTU is used, and in that, during the cleansing operation, the fixed bed is forced by the liquid pressure of the cleaning liquid against mechanically operating fragmenting means which separate the particles of the treating agent from one another and fluidize them to form a fluidized bed.

2. Process as claimed in claim 1, characterised in that the turbid liquid and the cleaning liquid consist predominantly of water.

3. Process as claimed in claim 1 or 2 characterised in that the fragmenting means are moved in such a way that they engage in the fixed bed and separate the particles of treating agent from one another.

4. Process as claimed in claims 1 to 3, characterised in that the aqueous cleaning liquid contains surface-active substances.

5. Process as claimed in claims 2 to 4, characterised in that before and after the aqueous cleaning liquid flows through the fixed bed there is an intermediate rinse with water.

6. Process as claimed in claims 1 to 5, characterised in that the turbid liquid used is a whey with a turbidity of more than 100 NTU and the treating agent used is an enzyme catalyst containing a lactase immobilised on carrier particles, and in that the process is used for hydrolysing the lactose in the whey.

7. Process as claimed in claims 6, characterised in that the aqueous cleaning liquid contains disinfectants.

8. Process as claimed in claims 6 or 7, characterised in that the aqueous cleaning liquid contains a dissolved proteolytic enzyme.

9. Process as claimed in claims 6 to 8, characterised in that an enzyme catalyst with a lactase activity of a least 100 U/g is used.

10. Fixed bed reactor for performing the process as claimed in claims 1 to 9, containing a reaction chamber arranged between two antechambers, this reaction chamber being at most half-filled with a fixed bed of the granular treating agent and being separated from the antechambers by perforations, the openings of which are smaller than the particles of the treating agent, and also inlet and outlet pipes which open into the antechambers, characterised in that the reaction chamber contains, outside the fixed bed, mechanically operating fragmenting means which are effective over a substantial proportion of the cross-section of the reaction chamber.

11. Fixed bed reactor as claimed in claim 10, characterised in that the fragmenting means consist of a lattice or grid of immovably mounted rods which are provided with points or blades directed towards the fixed bed.

12. Fixed bed reactor as claimed in claim 10, characterised in that the fragmenting means are movable in such a way that they can move at right angles to the longitudinal axis of the reactor by means of an external drive.

13. Fixed bed reactor as claimed in claim 10, characterised in that the fragmenting means consist of a rotatable and driveable shaft with projecting blades which is preferably mounted at right angles to the longitudinal axis of the reactor.

14. Fixed bed reactor as claimed in claims 10 to 13, characterised in that it contains, as the granular treating agent, an enzyme immobilised on carrier particles.

15. Enzymatic hydrolysis reactor for performing the process as claimed in claims 6 to 9, characterised in that the granular treating agent consists of an enzyme catalyst containing a lactase which is immobilised on carrier particles.

## Revendications

1. Procédé pour le traitement d'un liquide trouble contenant des matières solides finement divisées, avec un agent de traitement granulaire qui est disposé en un lit fixe traversé par le liquide trouble, la traversée du lit fixe par le liquide trouble étant interrompue périodiquement, un liquide de nettoyage étant envoyé à travers le lit fixe, à contre-courant par rapport à la direction du courant précédent du liquide trouble, pour débarrasser l'agent de traitement granulaire de matières solides finement divisées qui proviennent du liquide trouble et les particules de l'agent de traitement granulaire étant fluidisées et nettoyées à l'état de lit fluidisé, caractérisé en ce que l'on traite un liquide trouble ayant une turbidité de plus de 10 NTU (unités de turbidité néphélométriques) et en ce que, lors du nettoyage, le lit fixe est pressé par la pression du liquide de nettoyage contre des moyens de fragmentation qui agissent mécaniquement, ce qui fait que les particules de l'agent de traitement sont séparées les unes des autres et qu'elles sont fluidisées en un lit fluidisé.

2. Procédé selon la revendication 1, caractérisé en ce que le liquide trouble et le liquide de nettoyage se composent principalement d'eau.

3. Procédé selon la revendication 1 ou 2, caractérisé en ce que les moyens de fragmentation sont animés d'un mouvement tel qu'ils pénètrent dans le lit fixe et qu'ils séparent les unes des autres les particules de l'agent de traitement.

4. Procédé selon l'une quelconque des revendications 1 à 3, caractérisé en ce que le liquide aqueux de nettoyage contient des substances tensio-actives.

5. Procédé selon l'une quelconque des revendications 2 à 4, caractérisé en ce qu'avant et après la traversée du lit fixe par le liquide aqueux de nettoyage, il est effectué un rinçage intermédiaire à l'eau.

6. Procédé selon l'une quelconque des revendications 1 à 5, caractérisé en ce qu'il est utilisé, en tant que liquide trouble, un petit-lait ayant une turbidité de plus de 100 NTU et, en tant qu'agent de traitement, un catalyseur enzymatique qui contient une lactase, et qui est immobilisée sur des particules de support, et en ce que le procédé sert à l'hydrolyse du lactose dans le petit-lait.

7. Procédé selon la revendication 6, caractérisé en ce que le liquide aqueux de nettoyage contient un produit désinfectant.

8. Procédé selon la revendication 6 ou 7, caractérisé en ce que le liquide aqueux de nettoyage contient un enzyme protéolytique en solution.

9. Procédé selon l'une quelconque des revendications 6 à 8, caractérisé en ce qu'on utilise un catalyseur enzymatique qui présente une activité de lactase d'au moins 100 U/g.

10. Réacteur à lit fixe pour l'exécution du procédé selon l'une quelconque des revendications 1 à 9, contenant une zone de réaction qui est disposée entre deux pré-chambres, qui est remplie au maximum jusqu'à la moitié d'un lit fixe de l'agent de traitement granulaire et qui est séparée des pré-chambres par des fonds perforés dont les ouvertures sont plus petites que les particules de l'agent de traitement, ainsi que des conduites d'arrivée et de départ qui débouchent dans les pré-chambres, caractérisé en ce qu'il est disposé dans la zone de réaction, à l'extérieur du lit fixe, des moyens de fragmentation à action mécanique qui agissent sur la majeure partie de la section transversale de la zone de réaction.

11. Réacteur à lit fixe selon la revendication 10, caractérisé en ce que les moyens de fragmentation sont constitués par un treillis ou grille de barres montées en position immobile, qui sont équipées de pointes ou de lames en direction du lit fixe.

12. Réacteur à lit fixe selon la revendication 10, caractérisé en ce que les moyens de fragmentation sont mobiles de telle manière qu'ils puissent effectuer, sous l'action d'un mécanisme de commande extérieur, un mouvement dirigé perpendiculairement à l'axe longitudinal du réacteur.

13. Réacteur à lit fixe selon la revendication 10, caractérisé en ce que les moyens de fragmentation sont constitués par un arbre muni d'ailettes espacées, qui peut tourner et être entraîné et qui est disposé de préférence perpendiculairement à l'axe longitudinal du réacteur.

14. Réacteur à lit fixe selon l'une quelconque des revendications 10 à 13, caractérisé en ce qu'il contient, en tant qu'agent de traitement granulaire, un enzyme immobilisé sur des particules de support.

15. Réacteur d'hydrolyse enzymatique pour l'exécution du procédé selon l'une quelconque des revendications 6 à 9, caractérisé en ce que l'agent de traitement granulaire est constitué par un catalyseur enzymatique, contenant une lactase, qui est immobilisée sur des particules de support.

Figur 1

1   = Reaktor
2   = Festbett
3,4 = Siebböden
5,6 = Vorkammern
7   = freies Volumen

8,9 = Zuleitungen
10  = mechanische Zerteiler
11  = Welle
12  = abstehende Flügel
13  = Antrieb